# EUROPEAN PATENT APPLICATION

(11) **EP 4 156 089 A1**
(43) Date of publication of application: **29.03.2023**
(21) Application number: 21198541.1
(22) Date of filing: 23.09.2021
(51) Int. Cl.: G06T 7/00

(54) **METHOD, DEVICE AND SYSTEM FOR AUTOMATED PROCESSING OF MEDICAL IMAGES TO OUTPUT ALERTS FOR DETECTED DISSIMILARITIES**

(71) Applicant: Siemens Healthcare GmbH, 91052 Erlangen (DE)
(72) Inventor: Yoshihisa, SHINAGAWA, Downingtown, 19335 (US)
(74) Representative: Horn Kleimann Waitzhofer Patentanwälte PartG mbB

(57) **Abstract**

A method, device and system for automated processing of medical images to output alerts (A) for detected dissimilarities in the medical images is provided. In one aspect, the method comprises: receiving (301, 401) a first medical image of an anatomical object of a patient, the first medical image being acquired at a first instance of time, receiving (302, 402) a second medical image of the anatomical object of the patient, the second medical image being acquired at a second instance of time, determining (303, 405) a dissimilarity between the received first medical image and the received second medical image using a trained machine learning network, the trained machine learning network being adapted to determine two medical images having differences besides posture deformation, motion, variations of anatomy and scanning parameters as dissimilar and two medical images having no difference or only differences due to posture deformation, motion, variations of anatomy and scanning parameters as similar, and outputting (304, 406) a third medical image being generated based on the received first medical image and/or the received second medical image, said third medical image including an alert (A) for each determined dissimilarity, said alert (A) visualizing the determined dissimilarity between the received first medical image and the received second medical image in the third medical image.

## Description

The present invention relates to a computer-implemented method, to a computer-implemented device and to a system for automated processing of medical images to output alerts for detected dissimilarities in the medical images.

For detecting dissimilarities in medical images, for example for detecting a change in medical images of a pair of prior and current chest volumes, radiologists routinely read follow-up cases, e.g., a sequence of medical images of a certain anatomical object of a patient captured over a certain time period, for example two years. The radiologists compare prior studies and a current study, by comparing the corresponding medical images, to report interval changes and provide a new diagnosis, if any.

A first disadvantage of this conventional method is that it is cumbersome to compare two sets of images side by side, with one coming from the prior study and the other one from the current study, because the radiologists have to move their eye focus frequently between them. A second disadvantage is that doing so has not a very high accuracy in detecting dissimilarities, for example changes in the lung of a patient. A third disadvantage is that this conventional method is very time-consuming.

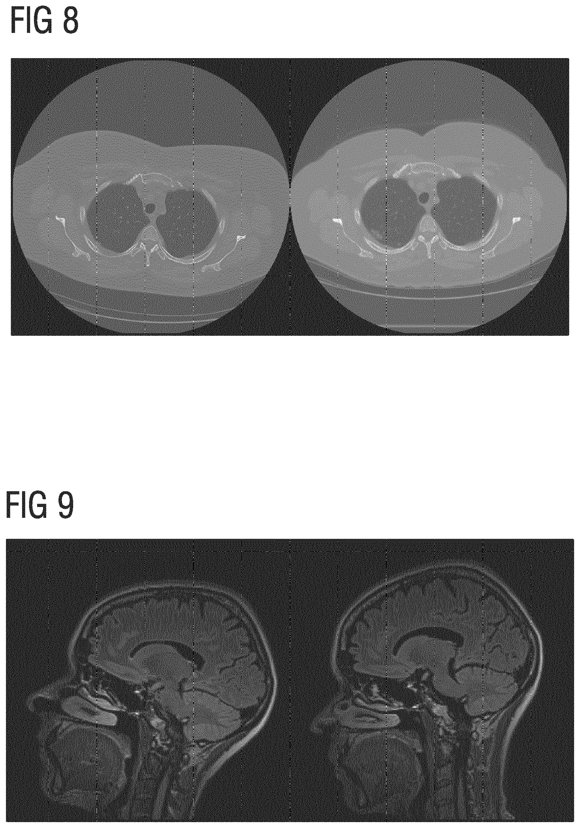

In this regard, FIG. 8 shows an example of conventional medical images showing a set of corresponding slices of a pair of prior and current chest volumes. For example, there is new nodule appearing in the right lung as shown in FIG. 8. Often times, due to the differences of postures and motions of organs, changes are not obvious to radiologists. Radiologists need to carefully compare in order not to miss changes. For example, one of the changes as shown in FIG. 9 around the sella turcica is not easily noticed due to the difference in the posture of the head.

Therefore, there is a need for a method and device which enables accurate and fast detection of dissimilarities in medical images.

There are conventional techniques for detecting changes between volumes by first registering the two volumes and then subtracting the prior from the current, i.e., the two volumes are brought to the same posture by applying rigid or deformable transformations to them and subtract them. These conventional techniques are useful to detect subtle changes and analyze the changes in detail, such as the growth of tumors. However, these conventional techniques are susceptible to noise and motion such as inspiration and/or expiration, and also scanning parameter differences. Too often, too many changes are detected.

Examples for these conventional techniques are disclosed by documents US 2014/0294263 A1, US 6678399 B2, and US 2005/0165292 A1. In detail, US 2014/0294263 A1 describes a framework for facilitating synchronized image navigation. At least first and second medical images are received. A non-linear mapping between the first and second medical images is generated. A selection of a given location in the first medical image is received in response to a user's navigational operation. Without deforming the second medical image, a target location in the second medical image is determined by using the non-linear mapping. The target location corresponds to the given location in the first medical image. An optimized deformation-free view of the second medical image is generated based at least in part on the target location. While the user performs navigational operations on the first medical image, the framework repeatedly receives the selection of the given location, determines the target location using the non-linear mapping, and generates the optimized deformation-free view of the second medical image based at least in part on the target location.

Further, US 6678399 B2 describes a method for computerized detection of lung nodules in computer tomography images, by which mask images are created such that subtractions of the mask image from a targeted CT section image reveal or highlight small lung nodules in the target CT section. The mask image is created utilizing the targeted CT section image along with other CT section images generated from the same CT scan. Based on these other section CT images and the targeted CT section image, a mask image can be created that is very similar to the target CT section image, but without the presence of small lung nodules. When the mask image is subtracted from the targeted CT section image, the differences between the mask images and the CT section images reveal small lung nodules. The mask image may be created by linear interpolation or a morphological filtered image.

Moreover, US 20050165292 A1 describes a method and an apparatus for allowing determination of patient position change relative to an imaging device and/or allowing digital subtraction in an operative position. The apparatus can include devices for determining a position of a patient at various times and comparing the various positions of the patient. Further, a digital subtraction may be performed if the patient change is not above a threshold value and/or if motion correction can occur.

The object of the invention is therefore to provide a method, device and system that enables an improved detection of dissimilarities in medical images.

According to a first aspect, a computer-implemented method for automated processing of medical images to output alerts for detected dissimilarities in the medical images is proposed. The method comprises:
receiving a first medical image of an anatomical object of a patient, the first medical image being acquired at a first instance of time,
receiving a second medical image of the anatomical object of the patient, the second medical image being acquired at a second instance of time,
determining a dissimilarity between the received first medical image and the received second medical image using a trained machine learning algorithm, the trained machine learning network being adapted to determine two medical images having differences besides posture and motion, in particular besides posture, deformation, motion, variations of anatomy and scanning parameters, as dissimilar and two medical images having no difference or only differences due to posture and motion, in particular besides posture, deformation, motion, variations of anatomy and scanning parameters, as similar, and
outputting a third medical image being generated based on the received first medical image and/or the received second medical image, said third medical image including an alert for each determined dissimilarity, said alert visualizing the determined dissimilarity between the received first medical image and the received second medical image in the third medical image.

The medical images may be captured by and received from a medical imaging unit. The medical imaging unit may include, for example, but not limited to, magnetic resonance imaging device, computed tomography device, X-ray imaging device, ultrasound imaging device, etc. The medical images may be three-dimensional and/or related to a volume. The medical images may include one or more anatomical objects associated with a patient. The anatomical objects may be in particular one or more body parts associated with the patient that may have been imaged. The anatomical objects may include, but not be limited to, one or more imaged organs, such as the lung. The medical images may, for example, be in the form of an array of pixels or voxels. Such arrays of pixels or voxels may be representative of intensity, absorption or other parameter as a function of three-dimensional position, and may, for example, be obtained by suitable processing of measurement signals obtained by one or more of the above-mentioned medical imaging units. In particular, the medical image may include information associated with a region of interest for tumor detection. Alternatively, the medical images may be received from a medical database including a plurality of medical images.

By means of the present method, a fast and accurate detection for dissimilarities in medical images is provided. In particular by using said trained machine learning algorithm such as deep neural networks, the present method is unsusceptible for noise and motion. Moreover, only essential changes may be visualized by said alerts to the user, e.g. a radiologist.

According to an embodiment, the machine learning network is trained such that two medical images having differences besides posture, deformation, motion, variations of anatomy and scanning parameters are labeled as dissimilar medical images and two medical images having no difference or only differences due to posture, deformation, motion, variations of anatomy and scanning parameters are labeled as similar medical objects.

As the machine learning network is trained regarding the differentiation between similar and dissimilar medical objects, the present method is very unsusceptible regarding noise and motion.

According to a further embodiment, the step of determining a dissimilarity includes determining at least one dissimilarity between the received first medical image and the received second medical image at a certain location of the anatomical object.

In particular, the present step of determining includes determining a plurality of dissimilarities between the received first medical image and the received second medical image at a plurality of certain locations in the anatomical object. In particular, the plurality of locations may embody a defined matrix in the first and second medical images.

According to a further embodiment, the method comprises:
extracting a first slice depicting a certain section of the anatomical object from the first medical image,
extracting a second slice depicting the certain section of the anatomical object from the second medical image,
determining a dissimilarity between the extracted first slice and the extracted second slice using the trained machine learning network, the trained machine learning network being adapted to determine two slices having differences besides posture, deformation, motion, variations of anatomy and scanning parameters as dissimilar and two slice having no difference or only differences due to posture, deformation, motion, variations of anatomy and scanning parameters as similar, and
outputting a third slice being generated based on the extracted first slice and/or the extracted second slice, said third slice including an alert for each determined dissimilarity, said alert visualizing the determined dissimilarity between the extracted first slice and the extracted second slice in the further slice.

In particular, the respective slice is a 2D slice and is extracted at a certain axial plane of the anatomical object In particular, the first medical image was captured in the past and the second medical image is a current medical image for the same patient. As a result, the first slice is a former or older slice and the second slice is a current slice. Moreover, the third slice may be based on the second slice and may additionally include the at least one alert visualizing a determined dissimilarity between the older slice (first slice) and the current slice (second slice). Therefore, any essential change between the two slices, i.e. the older slice and the current slice, is automatically output to the user. In particular by using the present trained machine learning network, also dissimilarities or changes can be visualized to the users which could not be identified by the user manually.

According to a further embodiment, the machine learning network is trained such that slices having differences besides posture and motion are labeled as dissimilar slices and slices having no difference or only differences due to posture and motion are labeled as similar slices. The present embodiment is unsusceptible to noise and motion, even on a slice level of the medical images taken from the anatomical object.

According to a further embodiment, the step of determining a dissimilarity includes determining a dissimilarity between the extracted first slice and the extracted second slice at a certain location of the anatomical object.

According to a further embodiment, the step of determining a dissimilarity between the extracted first slice and the extracted second slice using the trained machine learning network includes:
converting the extracted first slice into a first input vector,
converting the extracted second slice into a second input vector,
inputting the first input vector and the second input vector into the trained machine learning network for determining a distance between the first input vector and the second input vector, and
identifying the first extracted slice and the second extracted slice as similar if the determined distance is smaller than a certain threshold, and identifying the first extracted slice and the second extracted slice as dissimilar if the determined distance is greater than or equal to the certain threshold.

For example, the machine learning network may include a plurality of layers. In particular, one layer may be an embedding layer which is configured to execute above-mentioned converting steps, i.e. converting the extracted first slice into a first input vector and converting the extracted second slice into a second input vector. Moreover, said plurality of layers may include a shared LSTM (long shirt term memory) and CNN (convolutional neural network) executing above-mentioned inputting step and above-mentioned identifying step. For example, the extracted first slice is converted into a first input vector h₁ and the extracted second slice is converted into a second input vector h₂. If the two slices are similar, the converted vectors h₁ and h₂ are similar, i.e. a distance ∥h₁ - h₂∥ is small. In other words, locations in the slices are detected where ∥h₁ - h₂∥ is large. Such locations contain changes that are not merely due to posture and motion.

According to a further embodiment, the step of determining a distance is executed for a plurality of different locations in the first slice and in the second slice, wherein a certain location in the first slice and in the second slice is identified as similar, if the determined distance is smaller than the certain threshold, and the certain location in the first slice and in the second slice is identified as dissimilar, if the determined distance is greater than or equal to the certain threshold.

According to a further embodiment, the step of determining a distance is executed for a plurality of different locations in the first slice and in the second slice, wherein a certain location in the first slice and the most similar slice among a plurality of the second slices is identified as similar, if the determined distance is smaller than the certain threshold, and the certain location in the first slice and in the second slice is identified as dissimilar, if the determined distance is greater than or equal to the certain threshold.

According to a further embodiment, an alert is allocated to a certain location if the certain location is identified as dissimilar. Thus, an alert may visualize any location-specific dissimilarity or change besides posture and motion to the user.

According to a further embodiment, the machine learning network is a neural network, in particular a convolutional neural network (CNN), a Siamese network or a triplet network.

According to a further embodiment, each of the first medical image, the second medical image and the third medical image is a 3D medical image of the anatomical object of the patient.

According to a further embodiment, each of the first slice, the second slice and the third slice is a 2D slice.

According to a further embodiment, the alert is visualized using a certain color, using augmented reality and/or using virtual reality.

According to a further embodiment, the first medical image and the second medical image are acquired by a medical imaging unit, in particular by a MR scanner or a CT scanner.

According to a further embodiment, the first instance of time and the second instance of time are different. For example, between the first instance of time and the second instance of time may be months or years.

According to a second aspect, a computer-implemented device for automated processing of medical images to output alerts for detected dissimilarities in medical images is proposed. The computer-implemented device comprises:
one or more processing units,
a receiving unit which is configured to receive one or more medical images captured by a medical imaging unit, and
a memory coupled to the one or more processing units, the memory comprising a module configured to perform the method steps of the first aspect or of any embodiment of the first aspect using a trained machine learning network.

The respective unit, e.g., the processing unit or the receiving unit, may be implemented in hardware and/or in software. If said unit is implemented in hardware, it may be embodied as a device, e.g., as a computer or as a processor or as a part of a system, e.g., a computer system. If said unit is implemented in software, it may be embodied as a computer program product, as a function, as a routine, as a program code or as an executable object.

The embodiments and features according to the first aspect are also embodiments of the second aspect.

According to a third aspect, a system for automated processing of medical images to output alerts for detected dissimilarities in medical images is proposed. The system comprising:
one or more servers,
a medical imaging unit coupled to the one or more servers,
the one or more servers comprising instructions, which when executed causes the one or more servers to perform the method steps of the first aspect or of any embodiment of the first aspect.

The embodiments and features according to the first aspect are also embodiments of the third aspect.

According to a fourth aspect, a computer program product is proposed, the computer program product comprising machine readable instructions, that when executed by one or more processing units, cause the one or more processing units to perform the method of the first aspect or of any embodiment of the first aspect.

The embodiments and features according to the first aspect are also embodiments of the fourth aspect.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

According to a fifth aspect, a computer readable medium is proposed on which program code sections of a computer program are saved, the program code sections being loadable into and/or executable in a system to make the system execute the method of the first aspect or of any embodiment of the first aspect when the program code sections are executed in the system.

The embodiments and features according to the first aspect are also embodiments of the fifth aspect.

The realization by a computer program product and/or a computer-readable medium has the advantage that already existing management systems can be easily adopted by software updates in order to work as proposed by the invention.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features, and advantages of the present invention will become apparent from the subsequent description and dependent claims, taken in conjunction with the accompanying drawings, in which:
- FIG. 1: illustrates a block diagram of a client-server architecture embodying a system for automated processing of medical images to output alerts for detected dissimilarities in the medical images according to an embodiment of the present invention,
- FIG. 2: illustrates a block diagram of a data processing system embodying a computer-implemented device for automated processing of medical im-ages to output alerts for detected dissimilarities in the medical images,
- FIG. 3: illustrates a flowchart of a first embodiment of a method for automated processing of medical images to output alerts for detected dissimilarities in the medical images,
- FIG. 4: illustrates a flowchart of a second embodiment of a method for automated processing of medical images to output alerts for detected dis-similarities in the medical images,
- FIG. 5: illustrates a flowchart of an embodiment of the step of determining a dissimilarity between an extracted first slice and an extracted second slice,
- FIG. 6: illustrates an example of a generated medical image including an alert visualizing a determined dissimilarity in medical images,
- FIG. 7: illustrates a further example of a generated medical image including an alert visualizing a determined dissimilarity in medical images,
- FIG. 8: illustrates an example of conventional medical images showing a set of corresponding slices of a pair of prior and current chest volumes, and
- FIG. 9: illustrates an example of conventional medical images showing a set of corresponding slices of a pair of prior and current brain volumes.

Hereinafter, embodiments for carrying out the present invention are described in detail. The various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

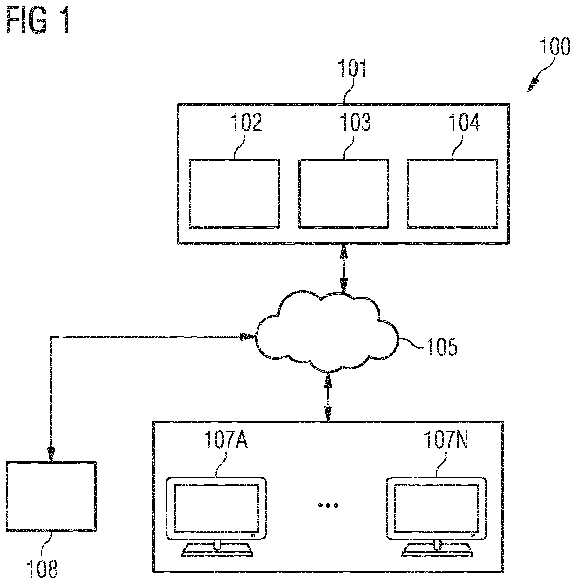

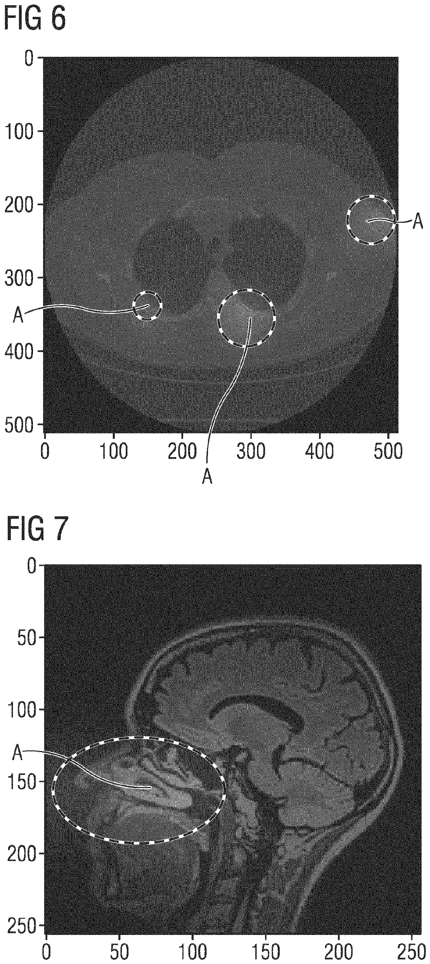

FIG. 1 provides an illustration of a block diagram of a client-server architecture embodying a system for automated processing of medical images to output alerts for detected dissimilarities in the medical images. The client-server architecture 100 comprises a server 101 and a plurality of client devices 107A-N. Each of the client devices 107A-N is connected to the server 101 via a network 105, for example, local area network (LAN), wide area network (WAN), WiFi, etc. In one embodiment, the server 101 is deployed in a cloud computing environment. As used herein, "cloud computing environment" refers to a processing environment comprising configurable computing physical and logical resources, for example, networks, servers, storage, applications, services, etc., and data distributed over the network 105, for example, the internet. The cloud computing environment provides on-demand network access to a shared pool of the configurable computing physical and logical resources. The server 101 may include a medical database 102 that comprises medical images and associated medical data related to a plurality of patients that is maintained by a healthcare service provider. In an embodiment, the medical database 102 comprises images captured by a MR scanner and/or by a CT scanner. The server 101 may include a module 103 that is configured to perform automated processing of medical images to output alerts A (see FIG. 6 and FIG. 7) for detected dissimilarities in medical images. Additionally, the server 101 may include a network interface 104 for communicating with the client device 107A-N via the network 105.

The client devices 107A-N are user devices, used by users, for example, medical personnel such as a radiologist, pathologist, physician, etc. In an embodiment, the user device 107A-N may be used by the user to receive medical images associated with the patient. The data can be accessed by the user via a graphical user interface of an end user web application on the user device 107A-N. In another embodiment, a request may be sent to the server 101 to access the medical images associated with the patient via the network 105. An imaging unit 108 may be connected to the server 101 through the network 105. The unit 108 may be a medical imaging unit 108 capable of acquiring a plurality of medical images. The medical imaging unit 108 may be, for example, a scanner unit such as a magnetic resonance imaging unit, computed tomography imaging unit, an X-ray fluoros-copy imaging unit, an ultrasound imaging unit, etc.

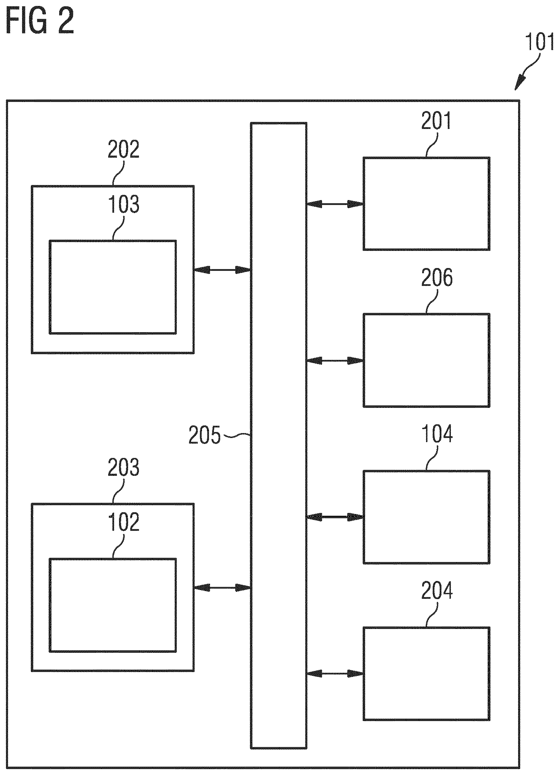

FIG. 2 is a block diagram of a data processing system 101 in which an embodiment can be implemented, for example, as a system 101 for automated processing of medical images to output alerts A for detected dissimilarities in the medical images, configured to perform the processes as described therein. It is appreciated that the server 101 is an exemplary implementation of the system in FIG. 2. In FIG. 2, said data processing system 101 comprises a processing unit 201, a memory 202, a storage unit 203, an input unit 204, an output unit 206, a bus 205, and a network interface 104.

The processing unit 201, as used herein, means any type of computational circuit, such as, but not limited to, a microprocessor, microcontroller, complex instruction set computing microprocessor, reduced instruction set computing microprocessor, very long instruction word microprocessor, explicitly parallel instruction computing microprocessor, graphics processor, digital signal processor, or any other type of processing circuit. The processing unit 101 may also include embedded controllers, such as generic or programmable logic devices or arrays, application specific integrated circuits, single-chip computers, and the like.

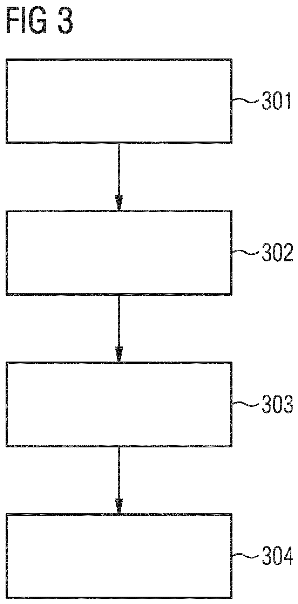

The memory 202 may be volatile memory and non-volatile memory. The memory 202 may be coupled for communication with said processing unit 201. The processing unit 201 may execute instructions and/or code stored in the memory 202. A variety of computer-readable storage media may be stored in and accessed from said memory 202. The memory 202 may include any suitable elements for storing data and machine-readable instructions, such as read only memory, random access memory, erasable programmable read only memory, electrically erasable programmable read only memory, a hard drive, a removable media drive for handling compact disks, digital video disks, diskettes, magnetic tape cartridges, memory cards, and the like. In the present embodiment, the memory 201 comprises a module 103 stored in the form of machine-readable instructions on any of said above-mentioned storage media and may be in communication to and executed by processing unit 201. When executed by the processing unit 201, the module 103 causes the processing unit 201 to automatically process medical images for outputting alerts A for detected dissimilarities in the medical images. Method steps executed by the processing unit 201 to achieve the abovementioned functionality are elaborated upon in detail in FIG. 3, 4, and 5.

The storage unit 203 may be a non-transitory storage medium which stores a medical database 102. The medical database 102 is a repository of medical images and associated medical data sets related to one or more patients that is maintained by a healthcare service provider. The input unit 204 may include input means such as keypad, touch-sensitive display, camera (such as a camera receiving gesture-based inputs), etc. capable of receiving input signal such as a medical image. The bus 205 acts as interconnect between the processor 201, the memory 202, the storage unit 203, the input unit 204, the output unit 206 and the network interface 104.

Those of ordinary skilled in the art will appreciate that said hardware depicted in FIG. 1 may vary for particular implementations. For example, other peripheral devices such as an optical disk drive and the like, Local Area Network (LAN)/ Wide Area Network (WAN)/ Wireless (e.g., Wi-Fi) adapter, graphics adapter, disk controller, input/output (I/O) adapter also may be used in addition or in place of the hardware depicted. Said depicted example is provided for the purpose of explanation only and is not meant to imply architectural limitations with respect to the present disclosure.

A data processing system 101 in accordance with an embodiment of the present disclosure may comprise an operating system employing a graphical user interface. Said operating system permits multiple display windows to be presented in the graphical user interface simultaneously with each display window providing an interface to a different application or to a different instance of the same application. A cursor in said graphical user interface may be manipulated by a user through a pointing device. The position of the cursor may be changed and/or an event such as clicking a mouse button, generated to actuate a desired response.

One of various commercial operating systems, such as a version of Microsoft Windows^{™}, a product of Microsoft Corporation located in Redmond, Washington may be employed if suitably modified. Said operating system is modified or created in accordance with the present disclosure as described. Disclosed embodiments provide systems and methods for processing medical images.

FIG. 3 illustrates a flowchart of a first embodiment of a method for automated processing of medical images to output alerts A (see FIG. 6 and FIG. 7, for example) for detected dissimilarities in the medical images. In particular, the method of FIG. 3 may be executed by above discussed module 103. The embodiment of FIG. 3 includes the method steps 301 - 304:
In step 301, a first medical image of an anatomical object of a patient is received, the first medical image being acquired by a medical imaging unit 108 at a first instance of time.

In step 302, a second medical image of the same anatomical object of the patient is received, the second medical image being acquired at a second instance of time. The first instance of time and the second instance of time are different. For example, the first medical image was captured in September 2020, and the second medical image was captured in September 2021. In particular, the first medical image and the second medical image are acquired by the medical imaging unit 108, in particular by an MR scanner or a CT scanner.

In step 303, a dissimilarity between the received first medical image and the received second medical is determined using a trained machine learning network. Determining a dissimilarity may include measuring or calculating the similarity between the two medical images. The machine learning network may be a neural network, in particular a convolutional neural network (CNN), a Siamese network or a triplet network. The trained machine learning network is adapted to determine two medical images having differences besides posture and motion as dissimilar and two medical images having no difference or only differences due to posture deformation, motion, variations of anatomy and scanning parameters as similar.

In the training phase, the machine learning network is trained such two medical images having differences besides posture and motion are labeled as dissimilar medical images and two medical images having no difference or only differences due to posture deformation, motion, variations of anatomy and scanning parameters are labeled as similar medical images.

In step 303, determining a dissimilarity may include determining at least one dissimilarity between the received first medical image and the received second medical image at a certain location of the anatomical object. In particular, for a plurality of corresponding locations in the first medical image and the second medical image, a similarity is measured or calculated for the corresponding locations of a pair of a prior medical image (first medical image) and a current medical image (second medical image).

In step 304, a third medical image being generated based on the received first medical image and/or received second medical image is output to a user, for example by one of the client devices 107A ― 107N as shown in FIG. 1. The output third medical image may include an alert A for each determined dissimilarity. For example, the medical image of FIG. 6 corresponding to a third medical image includes three alerts A visualizing three different dissimilarities. For example, the output third medical image may be based on the current medical image (second medical image) including the generated alerts A, three sections (illustrated by circles in the example of FIG. 6).

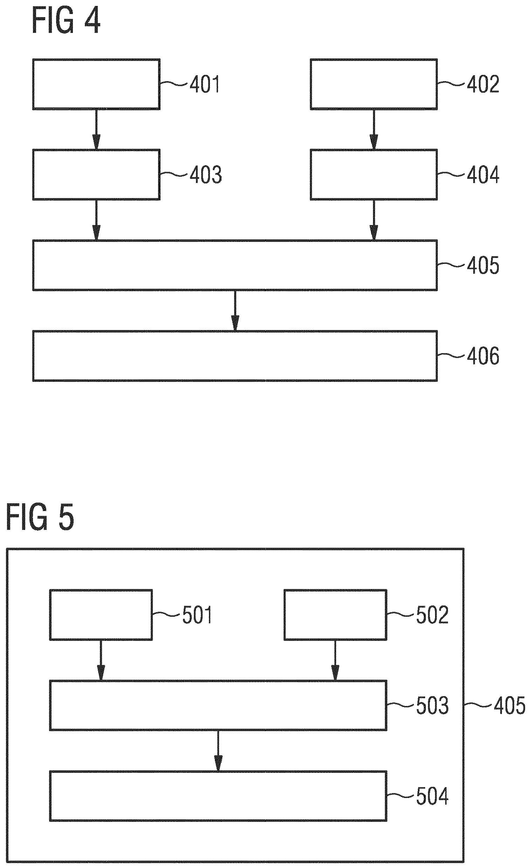

FIG. 4 illustrates a flowchart of a second embodiment of a method for automated processing of medical images to output alerts A for detected dissimilarities in the medical images. Also, the method of FIG. 4 may be executed by above discussed module 103.

The method of FIG. 4 comprises the following method steps 401 - 406:
In step 401, a first medical image of an anatomical object of a patient is received, the first medical image being acquired by a medical imaging unit 108 at a first instance of time.

In step 402, a second medical image of the same anatomical object of the same patient is received, the second medical image being acquired by the medical imaging unit 108 at a second instance of time. The first instance of time and the second instance of time are different to each other.

In step 403, a first slice depicting a certain section of the anatomical object is extracted from the first medical image.

In an analogous way, in step 404, a second slice corresponding to said first slice and depicting the same certain section of the same anatomical object is extracted from the second medical image. As a result, the first slice and the second slice are showing the same object or part of the same object, with changes in time as they are captured at different instances of time.

In step 405, a dissimilarity between the extracted first slice and the extracted second slice is determined using the trained machine learning network, the trained machine learning network being adapted to determine two slices having differences besides posture deformation, motion, variations of anatomy and scanning parameters as dissimilar and two slices having no difference or only differences due to posture in motion as similar.

In this regard, FIG. 5 shows an embodiment of the determining step 405 of FIG. 4. According to FIG. 5, determining a dissimilarity between the extracted first slice and the extracted second slice using the trained machine learning network includes the following method steps 501 ― 504:
In step 501, the extracted first slice is converted into a first input vector configured for the trained machine learning network.

In step 502, the extracted second slice is converted into a second input vector configured for the trained machine learning network.

In step 503, the first input vector and the second input vector are input into the trained machine learning network for determining a distance d between the first input vector and the second input vector.

In step 504, the first extracted slice and the second extracted slice are identified as similar, if the determined distance d is smaller than a certain threshold. In contrast, if the determined distance is greater than or equal to the certain threshold, the first extracted slice and the second extracted slice are identified as dissimilar.

For example, each of the two slices is converted to a respective input vector h₁ and h₂ by the neural network being an example for the trained machine learning network. The neural network may be implemented as a Siamese network or a triplet network. If the two slices are similar, the converted vectors h₁ and h₂ are similar, i.e., the distance ∥h₁ - h₂∥ is small. In particular, if locations in the slices are detected where ∥h₁ - h₂∥ is large, such locations contain changes that are not merely due to posture and motion. In the training phase, a label Y may be set 1, if the slices are annotated as similar, and Y = 0, if they are dissimilar.

Coming back to FIG. 4 and the following last step 406. In step 406, a third slice being generated based on the extracted first slice and/or the extracted second slice is output, for example by a client device 107A ― 107N as shown in FIG. 1. The third slice may be based on the current slice (second slice) and may include an alert A for each determined dissimilarity, said alert A visualizing the determined dissimilarity between the extracted first slice and the extracted second slice in the third slice.

The foregoing examples have been provided merely for the purpose of explanation and are in no way to be construed as limiting of the present invention disclosed herein. While the invention has been described with reference to various embodiments, it is understood that the words, which have been used herein, are words of description and illustration, rather than words of limitation. Further, although the invention has been described herein with reference to particular means, materials, and embodiments, the invention is not intended to be limited to the particulars disclosed herein, rather, the invention extends to all functionally equivalent structures, methods and uses, such as are within the scope of the appended claims. Those skilled in the art, having the benefit of the teachings of this specification, may effect numerous modifications thereto and changes may be made without departing from the scope and spirit of the invention in its aspects.

### REFRENCE SIGNS

- 100: system
- 101: computer-implemented device
- 102: medical database
- 103: module
- 104: network interface
- 105: network
- 107A- 107N: client device
- 108: medical imaging unit
- 201: processing unit
- 202: memory
- 203: storage unit
- 204: input unit
- 301 ― 304: method steps
- 401 - 406: method steps
- 501 ― 504: method steps
- A: alert

## Claims

1. A computer-implemented method for automated processing of medical images to output alerts (A) for detected dissimilarities in the medical images, the method comprising:
receiving (301, 401) a first medical image of an anatomical object of a patient, the first medical image being acquired at a first instance of time,
receiving (302, 402) a second medical image of the anatomical object of the patient, the second medical image being acquired at a second instance of time,
determining (303, 405) a dissimilarity between the received first medical image and the received second medical image using a trained machine learning algorithm, the trained machine learning network being adapted to determine two medical images having differences besides posture, deformation, motion, variations of anatomy and scanning parameters as dissimilar and two medical images having no difference or only differences due to posture, deformation, motion, variations of anatomy and scanning parameters as similar, and
outputting (304, 406) a third medical image being generated based on the received first medical image and/or the received second medical image, said third medical image including an alert (A) for each determined dissimilarity, said alert (A) visualizing the determined dissimilarity between the received first medical image and the received second medical image in the third medical image.

2. The computer-implemented method of claim 1,
wherein the machine learning algorithm is trained such that two medical images having differences besides posture and motion are labeled as dissimilar medical images and two medical images having no difference or only differences due to posture, deformation, motion, variations of anatomy and scanning parameters are labeled as similar medical objects.

3. The computer-implemented method of claim 1 or 2,
wherein the step of determining (303) a dissimilarity includes determining at least one dissimilarity between the received first medical image and the received second medical image at a certain location of the anatomical object.

4. The computer-implemented method of one of claims 1 to 3, comprising
extracting (403) a first slice depicting a certain section of the anatomical object from the first medical image,
extracting (404) a second slice depicting the certain section of the anatomical object from the second medical image,
determining (405) a dissimilarity between the extracted first slice and the extracted second slice using the trained machine learning network, the trained machine learning network being adapted to determine two slices having differences besides posture, deformation, motion, variations of anatomy and scanning parameters as dissimilar and two slices having no difference or only differences due to posture, deformation, motion, variations of anatomy and scanning parameters as similar, and
outputting (406) a third slice being generated based on the extracted first slice and/or the extracted second slice, said third slice including an alert for each determined dissimilarity, said alert (A) visualizing the determined dissimilarity between the extracted first slice and the extracted second slice in the further slice.

5. The computer-implemented method of claim 4,
wherein the machine learning algorithm is trained such that slices having differences besides posture and motion are labeled as dissimilar slices and slices having no difference or only differences due to posture, deformation, motion, variations of anatomy and scanning parameters are labeled as similar slices.

6. The computer-implemented method of claim 4 or 5,
wherein the step of determining (405) a dissimilarity includes determining a dissimilarity between the extracted first slice and the extracted second slice at a certain location of the anatomical object.

7. The computer-implemented method of claim 5 or 6,
wherein the step of determining (405) a dissimilarity between the extracted first slice and the extracted second slice using the trained machine learning algorithm includes:
converting (501) the extracted first slice into a first input vector,
converting (502) the extracted second slice into a second input vector,
inputting (503) the first input vector and the second input vector into the trained machine learning network for determining a distance between the first input vector and the second input vector, and
identifying (504) the first extracted slice and the second extracted slice as similar if the determined distance is smaller than a certain threshold, and identifying the first extracted slice and the second extracted slice as dissimilar if the determined distance is greater than or equal to the certain threshold.

8. The computer-implemented method of claim 7,
wherein the step (503) of determining a distance is executed for a plurality of different locations in the first slice and in the second slice,
wherein a certain location in the first slice and in the second slice is identified as similar, if the determined distance is smaller than the certain threshold, and the certain location in the first slice and in the second slice is identified as dissimilar, if the determined distance is greater than or equal to the certain threshold.

9. The computer-implemented method of claim 8,
wherein an alert (A) is allocated to a certain location if the certain location is identified as dissimilar.

10. The computer-implemented method of one of claims 1 to 9,
wherein the machine learning algorithm is a neural network, in particular a convolutional neural network (CNN), a Siamese network or a triplet network.

11. The computer-implemented method of one of claims 1 to 10,
wherein the alert (A) is visualized using a certain color, using augmented reality and/or using virtual reality.

12. A computer-implemented device (101) for automated processing of medical images to output alerts (A) for detected dissimilarities in medical images, the computer-implemented device (101) comprising:
one or more processing units (201),
a receiving unit (204) which is configured to receive one or more medical images captured by a medical imaging unit (108), and
a memory (202) coupled to the one or more processing units (201), the memory (202) comprising a module (103) configured to perform the method steps as claimed in any one of claims 1 to 11 using a trained machine learning network.

13. A system (100) for automated processing of medical images to output alerts (A) for detected dissimilarities in medical images, the system (100) comprising:
one or more servers (101),
a medical imaging unit (108) coupled to the one or more servers (101), the one or more servers (101) comprising instructions, which when executed causes the one or more servers (101) to perform the method steps as claimed in any one of claims 1 to 11 using a trained machine learning network.

14. A computer program product comprising machine readable instructions, that when executed by one or more processing units (201), cause the one or more processing units (201) to perform method steps according to claims 1 to 11.

15. A computer readable medium on which program code sections of a computer program are saved, the program code sections being loadable into and/or executable in a system (100) to make the system (100) execute the method steps according to any one of the claims 1 to 11 when the program code sections are executed in the system (100).
